# EUROPEAN PATENT APPLICATION

(11) **EP 2 210 952 A1**
(43) Date of publication of application: **28.07.2010**
(21) Application number: 09151468.7
(22) Date of filing: 27.01.2009
(51) Int. Cl.: C12P 1/02

(54) **Process for the production of a compound or a composition employing a culture of microorganisms under circadian cultivation conditions**

(71) Applicant: Rijksuniversiteit Groningen, 9712 CP Groningen (NL)
(72) Inventor: Merrow, Martha Wolcott, 9718 KB, Groningen (NL)
(74) Representative: Hatzmann, Martin

(57) **Abstract**

The present invention relates to a process for the production of a compound or composition by using microorganisms comprising providing a microorganism capable of producing said compound or composition in cultivation medium that supports the production of said compound or composition by said microorganism, culturing said microorganism under a first cultivation condition for a period of between 8-30 hours, culturing said microorganism under a second cultivation condition for a period of between 8-30 hours; alternating cultivation between the first and second condition to generate a culture that exhibits a rhythmic production with circadian periodicity with respect to said compound or composition; arresting the cultivation of said microorganism during or after said rhythmic production, and recovering said compound or composition from said culture.

## Description

### FIELD OF THE INVENTION

The present invention is in the field of production of compounds or compositions by using microorganisms. The invention provides processes for the production of a compound or composition by using microorganisms.

### BACKGROUND OF THE INVENTION

Processes for the production of compounds or compositions by using microorganisms generally involve well-controlled cultivation conditions. A large number of fermented foods are produced under conditions that are either optimal for the growth of the microorganism or optimal for the development of specific flavours. For instance it is known that for the production of certain beers slow alcoholic fermentation under low temperatures is essential for obtaining the required product characteristics. Cheeses such as taleggio and parmigiano reggiano are often left to mature in caves as caves have a constant humidity and temperature.

For the production of for instance penicillin by *Penicillium chrysogenum,* maximum production yields are achieved when cultivation temperature and initial pH are kept at 25±1°C and 4, respectively. Such optimizations are well known to provide for increases in yield by an order of magnitude. In general and in fact, optimization of the production of compounds and compositions by microorganisms is invariably aimed at optimizing each individual parameter of the external environment that affects the performance of the microorganism, all of which external environmental parameters are collectively referred to as the cultivation conditions. Most optimizations are described determined incrementally (e.g., temperature for optimal production) and are subsequently implemented into the process as 'constant conditions'.

It has recently become apparent that microorganisms, similar to plants and animals, have evolved adaptive regulatory pathways that control biological functions in response to the changing external environment. The metabolic rhythms in yeast having a cycle of about 40 min to about 4-5 h are well described but there is no known environmental cycle that has shaped these rhythms. In higher organisms circadian (from the Latin circa diem, "about a day") rhythms are well-established constituting self-sustained cycles of gene expression, metabolic flux, physiological processes and behavioral activities varying with a periodicity of roughly 24 hours. These rhythms have been demonstrated in a prokaryote, *Synechococcus,* to contribute to fitness and survival.

There is an abundance of information concerning circadian rhythms in animals. Examples are known from zebrafish and nematodes to mammals. In the animals mechanistic details are known from the identification of clock genes to the desription of rhythmic, daily secretion of the hormone melatonin by the pineal gland of the hypothalamus in humans. The breadth of knowledge concerning rhythms in animals, and even in plants, contrasts the dearth of information concerning circadian rhythms in microbes, where the presence of rhythms with a ∼24 h periodicity is established in only a few representatives. The identification of circadian rhythms in microorganisms is at a relatively early stage.

Cyanobacteria are the most primitive organisms in which circadian rhythms have been clearly documented. Over the past 15 years, many circadian rhythms including rhythms of photosynthetic activity, nitrogen fixation, global gene expression, and cell division have been found in several cyanobacterial species. Among photosynthetic organisms, our knowledge of clock components and interactions is most highly advanced in the unicellular cyanobacterium *Synechococcus elongatus.* In this cyanobacterium, cell-division is regulated by a circadian clock and even when cell division is arrested by gene disruption, non-dividing cells still exhibit robust circadian rhythms of gene expression (Mori and Johnson. 2001. Journal of Bacteriology 183(8):2439-44). In the multicellular/syncitial filamentous fungus, *Neurospora crassa,* a circadian rhythm in asexual spore formation has allowed characterization of circadian rhythms and their regulating clock genes. The synchronization of these daily rhythms by circa-24h light and temperature cycles has been extensively studied in *Neurospora*. A lone report of atypical circadian rhythms (they showed abnormal sunchronization patterns) in the economically and medically important filamentous fungus *Apsergillus* (ref), has never been followed up. Decades ago, circadian rhythms in the green yeast, *Chlamydomonas rheinhardtii,* were demonstraed. This has recently been revisited, with the application of reporter gene techonology to elucidate clock genes in this microbe (refs). It should be noted that *Chlamydomonas* resembles plants and animals more than fungi when compared at the genome level. In non-photosynthetic microorganisms such as yeast ultradian rhythms have been reported, but circadian rhythms in unicellular, non-photosynthetic microorganisms have hitherto not received any attention.

### SUMMARY OF THE INVENTION

The present inventors have now discovered that cells cultivated under a circadian cycle develop a circadian rhythm in their metabolism, which rhythm is maintained upon termination of the cycle for at least one circadian period. More importantly, the present inventors discovered that this circadian rhythm coincides with periodic changes in the profile of metabolic products produced and the yield of individual compounds formed.

Thus, the present inventors have discovered that the amount of individual compounds formed and their amounts relative to one another are greatly influenced when maintaining the culture in a circa 24h environmental cycle and furthermore by the time within that cycle when products are harvested. It is certain that the overall composition of the fermentation or cultivation product will be different if the culture grown in cycling conditions.

As a case in point, the inventors discovered that a beer produced by a yeast grown under a circadian rhythm has a taste that is distinct from a beer produced under normal, constant cultivation conditions. Moreover, it is contemplated herein that a microbial productwhich is produced by arresting the process at the peak of metabolic activity will be quite different from a microbial product produced by arresting the process at the trough of metabolic activity in the circadian cycle (i.e. 180 degrees out of phase, the chemical composition of the 'media' (microbial product) is quite different).

This surprising finding provides for novel and advantageous processes for the production of a compounds or compositions by using microorganisms.

In a first aspect, the present invention provides a process for the production of a compound or composition by using microorganisms comprising the steps of:
a) providing a microorganism capable of producing said compound or composition in cultivation medium that supports the production of said compound or composition by said microorganism;
b) culturing said microorganism under a first cultivation condition for a period of between 8-30 hours;
c) culturing said microorganism under a second cultivation condition for a period of between 8-30 hours;
d) alternating steps (b) and (c) to thereby generate a culture of said microorganism that exhibits a rhythmic production with circadian periodicity with respect to said compound or composition; and
e) arresting the cultivation of said microorganism during or at the end of said rhythmic production, and
f) recovering said compound or composition from said culture.

In a preferred embodiment, said rhythmic production with respect to said compound or composition is expressed as a circadian rhythm in the concentration of at least one metabolite in the cultivation medium, preferably, said at least one metabolite is said compound or is a component of said composition.

In another preferred embodiment of a process of the invention, said cultivation condition is selected from the group consisting of:
- the temperature of said culture,
- the exposure of said culture to radiation,
- the culture pH, and
- the flux and/or concentration of oxygen, CO₂, nutrients and/or growth substrate in said culture.

Even more preferably, the cultivation condition is the temperature of the culture.

In equally suitable alternative embodiments of a process of the invention, said compound or composition is secreted by said microorganism in the culture medium; said composition is the culture medium optionally including the microorganism, and/or said compound is a constituent of the cells of said microorganism.

In yet another preferred embodiment of a process of the invention, step f) comprises recovering said compound or composition from said culture medium or from the cells of said microorganism.

In yet another preferred embodiment of a process of the invention, first and/or second cultivation conditions comprise continuous culture conditions.

In yet another preferred embodiment of a process of the invention, microorganism is selected from the group consisting of fungi, bacteria and microalgae, preferably said microorganism is a yeast or a bacterium, most preferably a yeast.

In another embodiment, said microorganism is selected from the group consisting of *Saccharomyces ceruisiae, Streptomyces,* and *Bacillus subtilis.*

In another embodiment of said process, said compound is selected from the group consisting of an enzyme, a drug, a biosurfactant, a flavouring compound, a monomer for producing synthetic polymers, and a biofuel.

In another embodiment of said process, said composition is beer, wine, vinegar, soy sauce or rice wine.

In another embodiment of said process, said composition is a mixture of chemical variants of an antibiotic compound.

In another aspect, the invention provides a composition obtainable by the process of the present invention.

In a preferred embodiment of a composition of the present invention, the composition is a microbial fermentation product selected from the group consisting of miso, soy sauce, tofu, tempeh, beer, bread, sake, sourdough, rice wine, whisky, Vodka, silage, pickle, sauerkraut, wine, vinegar, cider, mead, cheese, kefir, quark, crème fraîche, yogurt, fish sauce, shrimp paste, salami and prosciutto having improved odor and/or taste characteristics.

In another preferred embodiment of a composition of the present invention, the composition is a mixture of chemical variants of an antibiotic compound and produced by a single culture.

### DESCRIPTION OF THE DRAWINGS

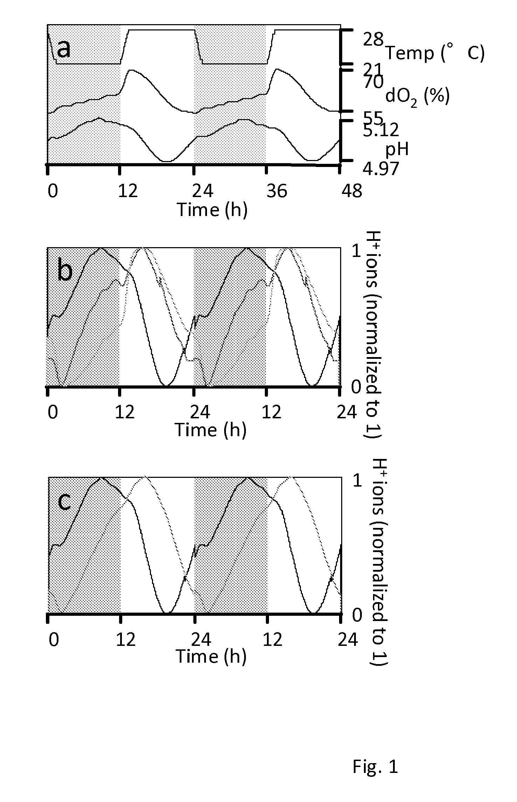

All examples pertain to *Saccharomyces cereuisiae* grown in a chemostat culture. Figure 1 illustrates that temperature cycles induce oscillations in dissolved O₂ and pH in media as described in the Examples. Grey panels represent cool temperature; open panels represent warm temperature. a) The experimental protocols used temperature cycles, shown here from 21°C to 28°C (upper tracing), which support oscillations in dissolved O₂ (middle tracing) and pH (lower tracing. b) In sub-24h cycles, the oscillations in H⁺ ion concentrations occur later within the temperature cycle. The solid line shows the H⁺ levels in a 24h oscillation, the stippled line shows the oscillation in a 23.5h temperature cycle and the dashed line portrays a 23h cycle. If the oscillations were simply a response to changing temperature, they would be expected to shift by the same amount of time as the temperature cycle. However, with a half hour change in the cycle length, the oscillation shifts back 4 - 6h within the cycle. This is a classic test (so-called Tcycles) for an endogenous circadian rhythm. c) Changing zeitgeber strength from 21°C to 28°C (solid line) to 18°C to 25°C (stippled line) shifts the entrained phase of the H⁺ ion oscillation. This is another test for circadian rhythms, namely changing the strength of the synchronizing agent, which often results in a change in synchronized phase.

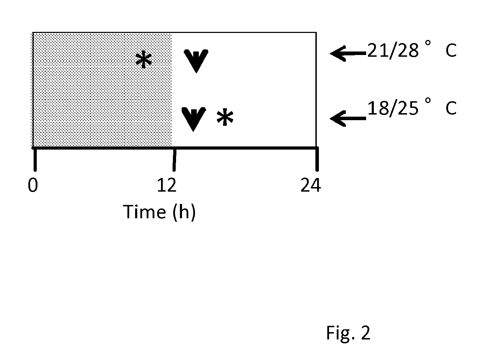

Figure 2 illustrates that the internal phase relationships change with zeitgeber strength. In lower temperature cycles (18°C to 25°C), the peak of the H⁺ oscillation moves into the warm phase, later than the peak of the dO₂. In warmer cycles (21°C to 28°C), the H⁺ oscillation occurs in the warm phase, earlier than the peak of the dO₂. If the oscillations were simply a reaction to the changing temperature cycles, the relationship between pH and O₂ would be expected to stay the same. If one of them moved within the cycle on a change in the mean temperature level, both would. Since they move differently, they are controlled by different processes. Presumably, the O₂ levels are driven in a straightforward way by the change up or down in temperature, whereas the pH oscillation is regulated by a circadian clock mechanism, in that is shows systematic phase relationships with the entraining cycle, as would a pendulum.

Figure 3 shows that the yeast chemostat culture behaves as a rapidly damping oscillator. A culture was entrained to a temperature cycle (12 h each at 21°C to 28°C) and released to warm temperature (28°C) constant conditions. The relative H⁺ ion concentration of the culture is shown.

Figure 4 illustrates that gene expression oscillates with the pH oscillation. MEP2 RNA was measured (3 experimental replicates) in cell extracts from free running cells in constant conditions. The dashed line shows the H+ ion oscillation; the solid line is MEP2 RNA normalized to actin RNA.

### DETAILED DESCRIPTION OF THE INVENTION

### Definitions

The term "microorganism", as used herein refers to a diverse group of minute, simple life forms that include archeae, bacteria, yeast, algae, fungi, and protozoa.

Suitable yeasts for use in a process of the invention include, but are not limited to, *Candida albicans, Candida boidinii, Candida utilis, Candida stellatoidea, Candida robusta, Candida sake, Candida claussenii, Candida rugosa, Hansenula minuta, Hansenula nonfermentans, Hansenula saturnus, Hansenula californica, Hansenula mrakii, Hansenula siluicola, Hansenula polymorpha, Hansenula wickerhamii, Hansenula capsulate, Hansenula glucozyma, Hansenula henricii, Kluyueromyces lactis, Kluyueromyces fragilis, Pichia farinose, Pichia polymorpha, Pichia membranefaciens, Pichia pinus, Pichia pastoris, Pichia trehalophila, Saccharomyces cereuisiae, Saccharomyces rosei, Saccharomyces bailii, Saccharomyces uvarum, Saccharomyces elegans, Saccharomyces rouxii, Torulopsis candida, Torulopsis bombicola, Torulopsis versatilis, Torulopsis glabrata, Torulopsis molishiana, Torulopsis nemodendra* and *Torulopsis nitratophila.*

Suitable bacteria for use in a process of the invention include, but are not limited to, *Arthrobacter parafficum, Arthrobacter simplex, Arthrobacter citreus, Aquifex pyrophilus, Bacillus subtilis, Bacillus cereus, Bacillus aureus, Bacillus circulans, Bacillus megaterium, Bacillus licheniformis, Bacillus sphaericus, Bacillus halodurans, Bacillus pumilus, Brevibacterium butanicum, Brevibacterium roseum, Brevibacterium flavum, Brevibacterium lactofermentum, Brevibacterium paraffinolyticum, Brevibacterium ketoglutamicum, Brevibacterium insectiphilium, Comamonas testosterone, Corynebacterium hydrocarbooxydans, Corynebacterium oleophilus, Corynebacterium hydrocarboclastus, Corynebacterium glutamicum, Corynebacterium viscosus, Corynebacterium dioxydans, Corynebacterium alkanum, Enterococcus gallinarum, Escherichia coli, Geobacillus stearothermophilus, Gluconobacter oxydans, Lactobacillus brevis, Lactobacillus bulgaricus, Lactobacillus acidophilus, Lactobacillus fermenti, Lactococcus lactis, Leuconostoc citrovorum, Leuconostoc bulgaricus, Leuconostoc dextranicum, Methanomonas methanica, and Methanomonas methanooxidans, Methylomonas methanolica, Microbacterium ammoniaphilum, Mycobacterium brevicale, Mycobacterium phlei, Mycobacterium rhodochrous, Nocardia coralline, Nocardia butanica, Nocardia salmonicolor, Pediococcus pentosaceus, Protaminobacter rubber, Pseudomonas methanolica, Pseudomonas fluorescens, Pseudomonas oleovorans, Pseudomonas putida, Pseudomonas boreopolis, Pseudomonas pyocinia, Pseudomonas methylphilus, Pseudomonas acidovorans, Pseudomonas aeruginosa, Pseudomonas striata, Rhodopseudomonas capsulatus, Salmonella typhimurium, Streptococcus cremoris, Streptococcus lactis, Streptococcus thermophilus, Thermus aquaticus* and *Vibrio cholerae.*

Suitable fungi for use in a process of the invention include, but are not limited to, *Aspergillus niger, Aspergillus glaucus, Aspergillus flavus, Aspergillus oryzae, Aspergillus terreus, Mucor mucedo, Mucor genevensis, Penicillium griseofulvum, Penicillium expansum, Penicillium digitatum, Penicillium italicum, Penicillium notatum, Penicillium chrysogenum, Rhizopus nigricans, Rhizopus oryzae, Rhizopus delemar, Rhizopus stolonifer,* and *Rhizopus arrhizus.*

The term "compound" as used herein refers to a chemical substance, in particular a biomolecule. Very suitably, the compound is selected from a peptide, a protein, an antibiotic, an alkanol (such as methanol, ethanol, propanol, (iso)butanol, pentanol and hexanol), an alkanediol (such as 1,3-propanediol), an amino acid (such a lysine, threonine, isoleucine, phenylalanine, tryptophan, aspartate, cysteine, and methionine), an organic acid (such as acetic acid, citric acid, lactic acid, succinic acid, 3-hydroxypropanoic acid, shikimic acid, acrylic acid), a vitamin (such as vitamin C, riboflavin), a fatty acid (such as butyric acid, hexanoic acid, caprylic acid, decanoic acid, lauric acid, myristic acid, palmitic acid, stearic acid, arachidic acid, behenic acid, lignoceric acid, α-linolenic acid, stearidonic acid, iicosapentaenoic acid, docosahexaenoic acid, linoleic acid, linolenic acid, arachidonic acid, oleic acid, erucic acid, and nervonic acid), a phospholipid (such as phosphatidylethanolamine, phosphatidylcholine, lechithin, phosphatidylserine, phosphatidylglycerol, phosphatidylinositol, and cerebroside) a sphingolipid (such as sphingomyelin, lysosphingomyelin, sphingosine, ceramide and phytosphingosine ), a glycolipid, a lipopeptide.

The compound is preferably not a hormone, serotonin, melatonin, (m)RNA, a corticosteroid, a glucocorticoid, a pheromone, cortisol, a cytokine, vasopressin, urine, pineal gland proteins, yeast fluorescent protein, non-excreted (intracellular) protein, bicarbonate, carbon monoxide, and carbon dioxide.

The term "metabolite" as used herein refers to a cellular chemical produced as part of metabolism, in particular of microbial origin.

The term "composition" as used herein refers to metabolic product profile or full range of metabolic products being secreted in the surrounding cultivation medium, optionally including the microbial cells that produced these products.

The terms "cultivation medium" and "culture medium" are used interchangeably herein and refer to a solid, semi solid or liquid substance containing essentially all nutrients and physical growth factors necessary for the growth, replication, maintenance, viability or activity of a microorganism being cultured. The term includes reference to a fresh culture medium in which no cell have grown, as well as to a "spent" culture medium from which the cells have been removed.

The term "culturing" as used herein refers to the growing microorganisms in a culture medium as well as to a process wherein the cells do not grow but assimilate, catabolise or convert substrates provided in the culture medium.

The term "cultivation condition" as used herein refers to conditions that are used to grow the microorganism for the production of compounds or compositions.

The term "cycle" as used herein is equivalent to the term rhythm and/or oscillation as used herein and refers to the period of a sinus or block oscillation when reference is made to the length of the cycle, and to any periodic fluctuation between two extreme values of a parameter of a cultivation condition in the context of the type of the cycle.

The term "alternating" as used herein refers to the fluctuations between the first and second cultivation condition.

The term "circadian rhythm" as used herein refers to a period of about 24 hours, but may in the context of the present invention be in the range of about 8 to about 60 hrs.

The term "zeitgeber" as used herein refers to any exogenous (external) cue, such as an environmental agent or event that entrains an organism's endogenous (internal) time-keeping system or biological clock. The term "entrain" as used herein refers to the process that results in the alignment of the period and phase of the circadian rhythm of the organism's metabolism with the period and phase of an external rhythm.

The term "growth" as used herein refers to replication, including genome duplication and cell division.

The term "metabolism" as used herein refers to the sum of the biochemical processes of a living cell.

The term "peak" as used herein refers to the maximum in the amplitude of a periodic fluctuation or crest (top of the sinus wave).

The term "trough" as used herein refers to the minimum in the amplitude of a periodic fluctuation (bottom of the wave).

The term "period" as used herein with reference to the length of the cycle is the time between two successive peaks or troughs.

The term "continuous culture" as used herein refers to system of growing microorganisms in a bioreactor which involves continuous operation where fresh culture medium is provided through an inlet at a rate such that the number of microorganisms in the culture equilibrates/is maintained at a constant level. The culture is harvested continuously through an outlet. A steady state, wherein the growth is essentially nutrient limited is generally attained upon 5 changes of the bioreactor volume.

### Description of the preferred embodiments

The present invention relates to a process for the production of a compound or composition by using microorganisms comprising the steps of providing a microorganism capable of producing said compound or composition in cultivation medium that supports the production of said compound or composition by said microorganism; culturing said microorganism under a first cultivation condition for a period of between 8-30 hours; culturing said microorganism under a second cultivation condition for a period of between 8-30 hours; alternating cultivation between said first and second cultivation condition to thereby generate a culture of said microorganism that exhibits a circadian rhythm with respect to growth or metabolism; the compound or compounds (the composition) can be harvested at an end stage (when the cells have metabolized their nutrition source), or in the cultivation of said microorganism when the metabolism of said microorganism is at a peak or at a trough with respect to the production of said compound or composition. The harvesting can involve collection of the growth media or it can involve extracting compond(s) from the cells.

The present inventors have developed a process to cultivate a microorganism under a circadian rhythm using a 24 hours temperature cycle of approximately a 6-18 hours high temperature (i.e. at a temperature of either 25 or 28°C) and a 18-6 hours low temperature (i.e. at a temperature of either 18 or 21°C. This growth leads to a synchronization of the metabolism of the microbial cells that are cultivated under this circadian rhythm to the length of the cycle. Metabolic cycles can assume the frequency of the cycle or they could also resonate with that frequency (multiply, giving more than one metabolic cycle per environmental cycle, or de-mulitply, giving less than one metabolic cycle per environmental cycle). Once synchronized, this rhythmic cycle in the metabolism of the culture results in a novel composition of metabolic products expressed in the cell and being secreted in the surrounding culture medium i.e. in a different metabolic product profile, that not only differs between the far ends of the metabolic response, but also when compared to the metabolic product profile of that same microorganism that is not cultivated under a circadian rhythm. The process of the invention can be applied for the production of microbial metabolic products in different ways, which is discussed below in more detail.

It has been suggested previously that yeast can grow in a circadian rhythm. For instance Edmunds (Edmunds, L.N., Jr, Cellular and molecular bases of biological clocks: Models and Mechanisms of circadian time keeping. (Springer, New York Heidelberg, 1988) describes that a circadian rhythm in cell division was observed in yeast grown under a 24 hour light/dark cycle. However, it cannot be excluded that in order for the authors to see the synchronization by light, the temperature of the culture was held at 12°C, which may presumably have slowed down cell division to about once per 24h. Extension of the protocol to temperatures other than 12°C has never been shown. Hence, whether a true circadian rhythm was indeed induced is not certain. Nonetheless, while recognizing the synchronization in cell division, it was hitherto never recognized that the yeast metabolism itself can be synchronized with a periodicity of 24 hours. This surprising finding led to the present invention of using such cultures for producing microbial metabolic products.

In a first aspect the present invention therefore provides a process for producing a microbial metabolic product comprising establishing in a microbial culture a circadian rhythm wherein the metabolism of the cells of said microbial culture is synchronized.

Investigations into the yeast metabolic cycle (YMC) are well documented (e.g. Tu et al., B. P. Tu et al. 2007. Proc Nat Acad Sci 104 (43), p.16886). It has been shown that yeast cells can synchronize their metabolic cycle in chemostat cultures under specified constant culture conditions. The cycle induced in this way comprises anywhere from a minutes-long to a 4-5 hours cycle. This short ultradian cycle, unlike the cycle contemplated in the present invention, is not induced (or maintained) by extracellular stimuli ("zietgebers"). It is to be expected that such ultradian cycles result in a different metabolic product profile (composition of metabolites secreted in the surrounding medium) when compared to the 24 hours circadian cycle.

A process of the invention comprises as a first step the provision of a microorganism capable of producing the desired compound or composition. When producing an antibiotic such as penicillin, the microorganism capable of producing said compound is for instance *Penicillium chrysogenum.* When producing a composition such as wine, the microorganism capable of producing said composition is for instance *Saccharomyces cerevisiae.* In addition, novel, hitherto unknown compounds, such as antibiotics, may be produced by microorganisms using the present invention.

The microorganism is provided in cultivation medium that supports the production of said compound or composition by said microorganism. With this, it is meant that in the case of *Penicillium chrysogenum* for producing penicillin a synthetic medium such as malt extract or potato-dextrose broth.

The medium may be a complex medium or a synthetic (defined) medium. Complex media as well as defined media are very suitable for the production of compositions according to the present invention. Examples of complex media include beans and bean-based broths (for the production of e.g. miso, soy sauce, tofu and tempeh), cereal doughs or cereal worts (for the production of e.g. beer, bread, sourdough, rice wine, whisky, Vodka), moist vegetable cuttings (for the production of e.g. pickle, sauerkraut and silage), fruit juices (for the production of e.g. wine, vinegar and cider), honey (for the production of e.g. mead), milk (for the production of e.g. cheese, kefir, quark, crème fraîche and yogurt), fish and crustaceans (for the production of e.g. fish sauce and shrimp paste), meat (for the production of e.g. salami and prosciutto), trypticase soy broth, or any other complex or synthetic medium such as for instance indicated in the Media Index of the USFDA online Bacteriological Analytical Manual (http://www.foodsafety.gov/∼ebam/bam-mi.html).

Essentially, a suitable medium for cultivation of said microorganism comprises a carbon source, a nitrogen source and a phosphorous source as sources of major nutrients and further optional sources of minor nutrients, such as co-factors, electrolytes and trace elements. As suitable carbon sources for industrial fermentation processes, glucose (derived from e.g. corn sugar, starch, or cellulose), sucrose (derived from e.g. sugarcane or sugar beet molasses), lactose (derived from e.g. milk whey), fats (derived from e.g. vegetable oils), or hydrocarbons (derived from e.g. petroleum fractions) may be used. As suitable nitrogen sources for industrial fermentation processes, protein (derived from e.g. soybean meal or cornsteep liquor), ammonia (as pure ammonia or ammonium salts), nitrate (as nitrate salts), or nitrogen (from e.g. air) may be used. As suitable phosphorous sources for industrial fermentation processes, phosphate salts are generally applicable.

The cultivation medium may be provided in a fermentor or bioreactor well known in the art and the microorganism can be added thereto to complete the first phase (step a) of the present invention.

A process of the present invention further comprises a second phase wherein the microorganism is cultured under cycling conditions rather than constant conditions. This cycle is imposed by cycling between at least two values of an environmental parameter. In essence, this can be achieved by culturing said microorganism under a first cultivation condition for a period of between 8-30 hours followed by culturing said microorganism under a second cultivation condition for a period of between 8-30 hours and continue alternating between the first and second condition for a selected period of time. Although it is scientifically not resolved how the culture successively enters in a metabolic rhythm, the fact is that upon the performance of between 2 to 20 cycles an oscillating metabolic activity pattern can be discerned in the culture, for instance in the rates in O₂ consumption or CO₂ production, or in hydrogen ion concentrations or in any of thousands of metabolites produced by cells as part of their normal biochemistry, indicating that the culture exhibits a rhythmic production with circadian periodicity with respect to said compound or composition. The term "with respect to said compound or composition" should be understood here as referring to the metabolic activity of the microorganism capable of producing said compound or composition in the cultivation medium that supports the production of said compound or composition by said microorganism under the prevailing cultivation conditions. The number of cycles to be imposed before an oscillating metabolic activity pattern can be discerned in the culture may vary between organisms, culture media, zeitgeber strength, etc. and is essentially a matter of optimization. In principle, an oscillation may already be entered after 1 cycle but this oscillation may be substantially less stable when compared to an oscillation induced by several or many cycles.

The cultivation conditions imposed are essentially fluctuated, alternated or cycled between a first and second condition, said second condition being different from said first, with a cycle of about 24 hours. Also suitable are cycles of 10-60 hrs, wherein for instance the first condition is maintained for 30 hrs and then changed to a second condition for 30 hrs, resulting in a total cycle period of 60 hrs. Although not strictly circadian, such cycles are contemplated within the context of the present invention. Also suitable are cycles
wherein the length of the first conditions is different from the length of the second condition. For instance 8 hrs light 16 hrs dark is a suitable cycle.

A circadian periodicity is state or condition characterized by a rhythmic or regular repetition in time or with an interval of about 24 hours. The rhythmic production with respect to said compound or composition is suitably expressed and observed as a circadian rhythm in the concentration of at least one metabolite in the cultivation medium. It is possible that said at least one metabolite is the desired compound produced by a process of the invention or is a component of the composition produced by a process of the invention. In that way, the production of the compound or the composition can be simultaneously be monitored when monitoring the circadian cycle.

The circadian rhythm is essentially imposed on the culture of the microorganism by subjecting that culture to a cycle in the magnitude, intensity or level of at least one environmental condition, i.e. cultivation condition, herein referred to as zeitgeber. Very suitable cultivation conditions that, when periodically cycled, result in a circadian rhythm include, but are not limited to, the temperature of the culture, the exposure of said culture to radiation, the culture pH, the flux and/or concentration of oxygen, CO₂, nutrients and/or growth substrate in said culture and (periodic) pharmacological manipulation. Very good results have been obtained by cycles in the temperature of the culture.

A cycle in the magnitude, intensity or level of at least one environmental parameter refers to a discrete rise or fall in the value of said parameter, or in a gradual increase or decrease therein.

For temperature, the cycle's amplitude may constitute a temperature difference of 1 to 50 °C, preferably 3-20 °C, more preferably 5-10°C between the first and second cultivation condition.

The microorganisms may be cultivated in cycles of temperature in a range close to or coinciding with the temperatures they may encounter in their natural habitat, winter or summer. For instance, a temperature range for microbial inhabitants of mammalian intestines may range from 5-37°C. However, in order to provide for more exotic microbial metabolic products, a temperature cycle may be chosen well outside the range that the microbe naturally encounters, but that is not lethal. It is expected that growth in extreme conditions will induce some combination of stress genes that will ultimately drive the expression of unique metabolic cocktails or product profiles.

For exposure to radiation, such as visible light, sun light, ultraviolet light or other forms of radiation, the cycle may constitute a difference in level of exposure to radiation of between 0 (no radiation) and a level that results in radiation damage (e.g. LD50 dosage). Preferably radiation refers to a cycle of light (as daylight or artificial light) and dark between the first and second cultivation condition.

Culture pH, when used to initiate the circadian rhythm, is preferably cycled between values that differ 1-4 pH units.

The flux or concentration of oxygen, CO₂, nutrients and/or growth substrate in the culture may also be used to initiate the circadian rhythm. In such cases, they are suitably cycled between values that differ about 1 and 3 orders of magnitude.

A process of the present invention further comprises a third phase
wherein the cultivation of the microorganism is arrested while said microorganism exhibits a circadian cycle with respect to said compound or composition. Arrest will generally involve the separation or removal of the cells from the culture medium. This may be performed by methods known per se, such as centrifugation or filtration. Alternatively, the cultivation can be arrested by rapidly decreasing the temperature of the culture such as freezing, by the addition of biocidals, by the addition of high concentrations of electrolytes such as Li, by a decrease or increase in pH, etc. The step of arresting the cultivation of the microorganism during the rhythmic production phase is aimed at preserving the production status with respect to the compound or composition.

A final step in the process of the present invention constitutes the recovery of the compound or composition from the culture. Recovery may entail isolation, purification, or mere collection. In a process for the production of a compound or composition by using microorganisms according to the present invention the compound or composition is preferably a secretion product from said microorganism, which product is secreted in the culture medium. Such compounds and compositions may be further isolated from the culture medium. Alternatively, the composition may constitute the culture medium (supernatant) *per se*, such as in the case of wine and beer. In that case, the composition refers to the culture medium itself having served as growth medium for said microorganism. Alternatively the composition refers to both the culture medium and the microorganism comprised therein. In yet another alternative embodiment, the compound or composition comprises one or more constituents of the cells of said microorganism. In such instances, the cells are generally harvested and the compounds or compositions may be isolated therefrom by methods known per se. Accordingly, a process for the production of a compound or composition according to the present invention comprises in step of f) the recovery of the compound or composition from the culture medium, or from the cells of the microorganism.

A process for the production of a compound or composition by the invention may be performed in (fed-) batch culture, or, preferably, in continuous culture (in essentially steady-state).

In principle any microorganism may be employed in a process of the present invention. Preferably, the microorganism is selected from the group consisting of fungi, bacteria and microalgae, preferably said microorganism is a yeast.

Most preferably the microorganism is selected from the group consisting of *Saccharomyces cervisiae*, a culture of one or more *Streptomyces* spp, and *Bacillus subtilis.*

The microorganism is preferably not a phototrophic microorganism (a microalgae or cyanobacterium).

The process of the present invention can be used for the production of a wide variety of compounds. Illustrative examples include enzymes, drugs, biosurfactants, flavouring compounds, monomers for the production of synthetic polymers, and biofuels. The compound of the present invention may be any bulk or fine chemical. Preferred compounds are selected from an enzyme, a drug, a biosurfactant, a flavouring compound, a monomer for producing synthetic polymers, and a biofuel.

The process of the present invention can be used for the production of a wide variety of compositions. The composition of the present invention may be any bean-, grain-, vegetable-, fruit-, honey-, dairy-, fish- or meat-based microbial fermentation product. Suitable fermentation product include miso, soy sauce, tofu, tempeh, beer, bread, sake, sourdough, rice wine, whisky, Vodka, pickle, sauerkraut, wine, vinegar, cider, mead, cheese, kefir, quark, crème fraîche, yogurt, fish sauce, shrimp paste, salami and prosciutto. Preferred examples include beer, wine, vinegar, soy sauce or sake.

The process of the present invention may also be used for the production of compositions comprising various chemical variants of specific compounds or of molecular variants within a group of compounds. Essentially, these can be produced by a single culture. The production of different chemical variants may yield completely new compounds which may exhibit altered activity relative to the original compound. Such a feature is of great interest to drug development and in particular to antibiotic discovery. Hence, compositions comprising a plurality of antibiotic compounds are specifically contemplated herein.

Also contemplated as part of the invention are compositions obtainable by the process of the invention. The particulars of such compositions are described herein above. The compositions of the present invention have an altered metabolic product profile which alteration can be detected by relatively straightforward analysis techniques well known in the art. The compositions may for instance have improved taste. Examples of compositions with altered metabolic product profile include beer, wine, vinegar, soy sauce or rice wine. In particular contemplated are such compositions exhibiting an improved taste or odour as determined by a human taste panel, compared to a control composition produced by the same microorganism under constant (conventional, not circadian cycling) cultivation conditions.

In another aspect, the present invention provides a process of improving the taste or odour characteristics of a microbial fermentation product selected from the group consisting of miso, soy sauce, tofu, tempeh, beer, bread, sake, sourdough, rice wine, whisky, Vodka, pickle, sauerkraut, wine, vinegar, cider, mead, cheese, kefir, quark, crème fraîche, yogurt, fish sauce, shrimp paste, salami and prosciutto comprising:
a) providing a microorganism capable of producing said fermentation product in cultivation medium that supports the production of said fermentation product by said microorganism;
b) culturing said microorganism under a first cultivation condition for a period of between 8-30 hours;
c) culturing said microorganism under a second cultivation condition for a period of between 8-30 hours;
d) alternating steps (b) and (c) to thereby generate a culture of said microorganism that exhibits a rhythmic production with circadian periodicity with respect to said compound or composition;
e) arresting the cultivation of said microorganism during said rhythmic production, and
f) recovering said compound or composition from said culture.

In another aspect, the present invention provides a microbial fermentation product selected from the group consisting of miso, soy sauce, tofu, tempeh, beer, bread, sake, sourdough, rice wine, whisky, Vodka, pickle, sauerkraut, wine, vinegar, cider, mead, cheese, kefir, quark, crème fraîche, yogurt, fish sauce, shrimp paste, salami and prosciutto having improved odor and taste characteristics produced according to the process describe above.

In another aspect, the present invention provides a process of increasing the number of chemical variants of an antibiotic compound produced by a microorganism comprising:
a) providing a microorganism capable of producing said antibiotic compound in cultivation medium that supports the production of said antibiotic compound by said microorganism;
b) culturing said microorganism under a first cultivation condition for a period of between 8-30 hours;
c) culturing said microorganism under a second cultivation condition for a period of between 8-30 hours;
d) alternating steps (b) and (c) to thereby generate a culture of said microorganism that exhibits a rhythmic production with circadian periodicity with respect to said compound or composition;
e) arresting the cultivation of said microorganism during or after said rhythmic production, and
f) recovering said compound or composition from said culture.

In another aspect, the present invention provides a mixture of chemical variants of an antibiotic compound produced according to the process describe above. The present invention also contemplates the use of the processes as disclosed herein for the industrial production of enzymes, and for the production of biofuel. It was for instance observed that during the production of beer, the rate of ethanol production was faster in circadian rhytmic culture conditions, compared to normal static culture conditions. Thus, the imposition of daily rhythms in temperature on certain cultures could improve efficiency of industrial fermentation.

Several utilities for the above-described process are indicated herein. One advantageous utility concerns the production of microbial fermentation products such as beer and other compositions described herein. Due to the circadian rhythm and the synchronization of the metabolic cycle, the metabolic composition is altered compared to the composition obtained when the microorganism is not cultivated under a circadian rhythm. The inventors have found that cultivating yeast under a circadian clock during the brewing process results in a different taste of the beer when compared to yeast that are cultivated under normal circumstances. In fact, the inventors have discovered a process for the production of a fermentation product that may be referred to as a "summer beer" (changing the cycle to 16 hours high temperature and 8 hours low temperature), or a "winter beer" (changing the cycle to 8 hours high temperature and 16 hours low temperature). This may also be used for wine making.

Another advantageous utility concerns the production of antibiotics (e.g. by *Streptomyces*). Without wishing to be bound by any theory, the present inventors hypothesize that the change in metabolic product profile upon cultivation of the microorganism under a circadian cycle that synchronizes metabolism has the effect that *inter alia* chemical variants of otherwise normal metabolites are produced. In the case of production of antibiotics by *Streptomyces*, this is believed to result in the release of chemical variants of the antibiotic(s) produced, and thus also potentially in the production of new antibiotics.

Yet another advantageous utility concerns the use of the process of the invention to develop new production strategies with microbial production strains such as *Bacillus subtilis. Bacillus subtilis* is widely used for the production of, for example, enzymes such as protopectinase, and surfactins (surface active agents exhibiting diverse biological activities including antiviral, antimycoplasmal, antitumoral, and antibacterial properties). Cultivation in cycling conditions presents potentially improved yields and/or purity of these compounds, as well as yielding novel compounds.

In another aspect, the present invention provides the use of a microbial composition produced by the process of the present invention (wherein said microorganism is cultivated under a circadian metabolic cycle) in a dedicated application, selected from amongst beer production, antibiotic production, enzyme production, etc. The invention will now be described in more detail in the following non-limiting Examples.

### EXAMPLES

Circadian timing is a fundamental biological process, underlying cellular physiology in animals, plants, fungi and the cyanobacteria. Circadian clocks organize gene expression, metabolism and behaviour such that they occur at appropriate times of day, or even times of year. Here, we use chemostat cultures to establish conditions that reveal characteristic clock properties as described in so many other species, thereby showing circadian timing in budding yeast. This opens the door for rapid progress into cellular clock mechanisms.

The circadian clock shares canonical properties amongst organisms from all phyla. One of these is a free running, circa-24h oscillation (circadian) in constant conditions. The phenomenon of self-sustained rhythmicity reflects the evolution of a daily timing system that developed in an environment that is utterly predictable in its alternation of light and darkness, higher and lower temperatures and numerous other qualities. The resulting system is robust enough to oscillate even in the absence of these external cues. Under natural condition, however, circadian rhythms are synchronised to environmental cycles (zeitgebers). The active process of synchronisation, called entrainment, results in the establishment of a stable phase relationship between the endogenous and exogenous rhythms that vary according to conditions such as strength or period (T) of the zeitgeber.

It is not known whether these features of circadian regulation exist in *S. cerevisiae*. In order to characterise clock properties in yeast, a chemostat culture system was developed that maintained cells in a stable environment over days to weeks. Using a minimal media and controlling pH levels, short, ultradian oscillations in metabolism and gene expression were reported in chemostat cultures (Tu et al. 2005 Science 310 (5751), 1152). The difference between the experiments of Tu *et al.* and the present invention is that Tu *et al.* use constant conditions (such as constant temperature and constant pH) to obtain ultradian rhythms, whereas the present invention uses circa-24h (or near-circadian) cycles.

The chemostats were subjected to temperature cycles with a period of 24 hours, to mimic a rhythmic environment (12h at 21°C and 12h at 28°C, unless otherwise specified). dO₂ in the media fluctuated with a period of 24h, reflecting daily alterations in metabolic rate (Fig. 1a). Under these conditions, we saw no ultradian oscillations. Similar to dO₂, daily rhythms in hydrogen ion concentration were also observed, with the pH of the incoming media (5.6) becoming 'conditioned' by the cells in the chemostat to oscillate at mean level of approximately pH 4.5. Net daily fluctuations corresponded to roughly 10⁶ H⁺ molecules/yeast cell/day.

One explanation for the observed oscillations in dO₂ and pH is that they arise from a metabolic switch that is thrown with each change in temperature rather than from an endogenous circadian oscillation. A switch-like mechanism would result in a simple yet systematic series of phase relationships between stimulus and response. The relationship between the dO₂, pH and temperature transitions would be consistent over a variety of conditions. In contrast, circadian entrainment should show a variety of phase relationships depending on the conditions. The cultures were subjected to cycles of shorter duration, which should lead to a later phase of entrainment if the observed oscillations were controlled by an endogenous timing system and not merely by the changing culture conditions. Consistent with circadian entrainment, the pH oscillation in the yeast culture shifted later with each shorter cycle (Fig. 1b). A 30 min decrease in cycle length resulted in a phase delay of 4 - 6h. Another feature of circadian systems is that their phase will change with increasing or decreasing zeitgeber strength. When the temperature cycle was shifted to a lower mean level, the phase of the pH rhythm shifted later within the cycle by 6h (Fig. 1c).

We probed the oscillations for other signs of robustness, which would distinguish circadian entrainment from a simple stimulus-response pattern that is driven by the temperature change. By varying conditions, we were able to uncouple the dO₂ and pH oscillations, so that they assumed different phase relationships (Fig. 2). Metabolic rate, as suggested by dO₂, was tied to temperature transitions, showing a peak about 2h into the warm incubation, while the peak in H⁺ ion concentration moved from the cold to the warm phase as the zeitgeber strength changed (by decreasing the mean temperature of the 24h cycle).

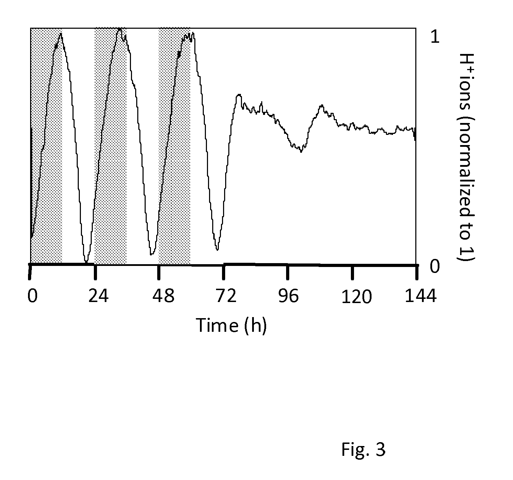

Systematic circadian entrainment (different phase relationships in different T-cycles and zeitgeber strengths) can be shown for robust, self-sustained, free running rhythms as well as for a weak oscillator that would rapidly damp in constant conditions. We investigated which state the yeast culture represented by releasing to constant conditions and monitoring H⁺ ion concentrations. The oscillation in pH continues for more than two cycles before damping out to a constant level (Fig. 3). This is a phenomenon that has been noted previously in microbial systems and in cell culture using mouse and rat fibroblasts and liver explants. Thus the yeast 24h timing mechanism is a damped oscillator, at least under these culture conditions. The rapid damping could either be due to loss of sustainment on the level of the cell or to an averaging effect of the community of cellular oscillators in the chemostat.

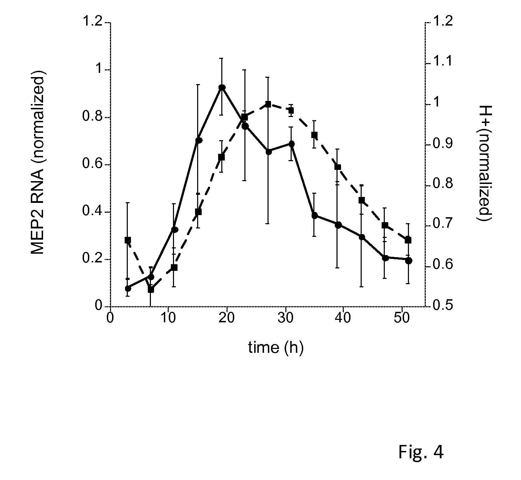

One of our goals is to elucidate molecular mechanisms of the circadian oscillator in yeast. So far, circadian clocks have been found to run either on a transcriptional-translational feedback loop (involving post-transcriptional processes), or on post-transcriptional processes or they can be a mixture of the two. We investigated gene transcription over the first 48h of constant conditions following 24h temperature cycles. To identify target genes, we considered that H⁺ trafficking suggests oscillations in output and/or intake of these molecules by the cells according to a circadian rhythm. One likely source of this biochemistry is regulation of nitrogen metabolism. As part of this process, ammonium is taken up via the MEP family of ammonium permeases. Active transport consequently removes H⁺ ions from the cell to prevent acidification. The MEP2 permease shows a high amplitude oscillation in gene expression in constant conditions with a period mirroring that of the pH oscillation (Fig. 4).

Although circadian clocks are found widely in nature, they have not been reported in *S. cerevisiae*, the most powerful genetic model system for cell biology. There is an extensive literature describing ultradian rhythms in yeasts (Tu et al. 2005 *supra*; Chance et al. 1965. J Biol Chem 240, 3170), and recently it was suggested that these short rhythms could be building blocks for longer circadian rhythms (Tu and McKnight. 2006. Nat Rev Mol Cell Biol 7 (9), 696). Although this is formally possible, we see no evidence for ultradian oscillations under the conditions used for these experiments. Several decades ago, experiments purported to show circadian rhythms of cell division in bulk cultures of yeast (Edmunds, L.N., Jr., ed., Cellular and molecular aspects of circadian oscillators: models and mechanisms for biological timekeeping. (Springer, Heidelberg, 1992), but these findings were never independently repeated. In the chemostat cultures described here, subjected to circadian temperature cycles, the cell division rate is approximately once per 9h and there is no obvious rhythm in cell division which could indicate gating of cell division to a specific time of day. Therefore, these temperature cycle protocols reveal distinct processes in *S. cerevisiae,* namely entrainment and a damped free-running rhythm, that are consistent with a circadian timing mechanism. Furthermore, we have shown clock-controlled molecular rhythms in gene expression of key metabolic and developmental pathways. These observations open the door for new approaches to elaborating circadian clock mechanisms and behaviours in eukaryotes.

### Materials and Methods

### Yeast strain and culture conditions.

The strain used throughout this study was *Saccharomyces cerevisiae* FY1679-2B (*MAT*α *ur*α*3-52 leu2*Δ*1 TRP1 his3*Δ*200 GAL2;* EUROSCARF, Frankfurt am Main, Germany; as described in Winston, et al. 1995 Yeast 11 (1), 53.

Inocula were prepared by transferring a single colony to a tube containing 15 ml YPD (1% Bacto-yeast extract, 2% Batco-peptone, and 2% glucose). Following overnight culture with shaking (200 rpm) at 25°C for 16 h, the cells were inoculated into 1 dm⁻³ of YPD and batch cultured at 30°C for approximately 36h. The end of the batch culture was identified as a rapid decrease in dO₂, after which time the culture was starved for an additional 4 h. Fermentors (APPLIKON, Schiedam, The Netherlands) were then operated in continuous mode. Sigma Antifoam A was used at 10 ml/L with an agitation rate was 750 rpm an aeration rate of 150 ml·min⁻¹. A working volume of 1 L was maintained with a dilution rate of about 0.09-0.1 h⁻¹ (unless otherwise specified). Dissolved oxygen (dO₂) and pH were measured every 2 min in the culture using an O₂ electrode and a pH sensor. Experiments were conducted in continuous dim light (Lumilux Interna, Osram).

### Zeitgeber cycles

Half of each cycle was spent in high temperature, the other half in low temperature. The temperature cycles were controlled by a circulating water bath (F25-ME, Julabo, Germany). All temperature transitions were programmed to occur over 60 minutes.

### RNA Preparation

Yeast cells were collected every 4 hours over 2 days of a free run, starting 2 h after the temperature transition from cold to warm. At each time point, 3.75 × 10⁸ cells per time interval were frozen in liquid nitrogen. Yeast total RNA was prepared using a slightly modified version of the hot phenol RNA extraction protocol (Schmitt et al. 1990. Nucleic Acids Res 18 (10), 3091.

The frozen yeast pellet was suspended in 400 µl AE buffer (50mM NaOAc pH5.3 and 10mM EDTA); 40 µl 10% SDS and 400 µl acidic phenol were added. The cells were disrupted by vortexing and then heated at 65°C for 30 min. The samples were cooled, centrifuged and the aqueous phase was reextracted with 400 µl acidic phenol followed by chloroform. RNA samples were purified and concentrated using NucleoSpin® RNA II Kit (MACHEREY-NAGEL GmbH & Co.).

### RT-PCR analysis

cDNA was prepared according to standard methods (ABI, xxx). 1 ul template cDNA was analysed in triplicate for each primer set. Primers were designed with Primer Express software (ABI). PCR reactions were performed according to standard methods (ABI).

### Data analysis

The output files from the fermenters were analyzed with CHRONO (Roenneberg & Taylor. Meth. Enzymol. 305, 104 (2000)). References

## Claims

1. A process for the production of a compound or composition by using microorganisms comprising the steps of:
a) providing a microorganism capable of producing said compound or composition in cultivation medium that supports the production of said compound or composition by said microorganism;
b) culturing said microorganism under a first cultivation condition for a period of between 8-30 hours;
c) culturing said microorganism under a second cultivation condition for a period of between 8-30 hours;
d) alternating steps (b) and (c) to thereby generate a culture of said microorganism that exhibits a rhythmic production with circadian periodicity with respect to said compound or composition; and
e) arresting the cultivation of said microorganism during or after said rhythmic production, and
f) recovering said compound or composition from said culture.

2. The process of claim 1, wherein said rhythmic production with respect to said compound or composition is expressed as a circadian rhythm in the concentration of at least one metabolite in the cultivation medium, preferably, said at least one metabolite is said compound or is a component of said composition.

3. The process of claim 1 or 2, wherein said cultivation condition is selected from the group consisting of:
- the temperature of said culture,
- the exposure of said culture to radiation,
- the culture pH, and
- the flux and/or concentration of oxygen, CO₂, nutrients and/or growth substrate in said culture,
preferably said cultivation condition being the temperature of said culture.

4. The process of any one of the preceding claims,
- wherein said compound or composition is secreted by said microorganism in the culture medium;
- wherein said composition is the culture medium optionally including the microorganism, or
- wherein said compound is a constituent of the cells of said microorganism.

5. The process of claim 4, wherein step f) comprises recovering said compound or composition from said culture medium or from the cells of said microorganism.

6. The process of any one of the preceding claims, wherein said first and/or second cultivation conditions comprise continuous culture conditions.

7. The process of any one of the preceding claims, wherein said microorganism is selected from the group consisting of fungi, bacteria and microalgae, preferably said microorganism is a yeast.

8. The process of any one of the preceding claims, wherein said microorganism is selected from the group consisting of *Saccharomyces cervisiae, Streptomyces*, and *Bacillus subtilis.*

9. The process of any one of the preceding claims, wherein said compound is selected from the group consisting of an enzyme, a drug, a biosurfactant, a flavouring compound, a monomer for producing synthetic polymers, and a biofuel.

10. The process of any one of the preceding claims, wherein said composition is beer, wine, vinegar, soy sauce or rice wine.

11. The process of any one of the preceding claims, wherein said composition is a mixture of chemical variants of an antibiotic compound.

12. Composition obtainable by the process of any one of the preceding claims.

13. Composition according to claim 12, wherein said composition is a microbial fermentation product selected from the group consisting of miso, soy sauce, tofu, tempeh, beer, bread, sake, sourdough, rice wine, whisky, Vodka, silage, pickle, sauerkraut, wine, vinegar, cider, mead, cheese, kefir, quark, crème fraîche, yogurt, fish sauce, shrimp paste, salami and prosciutto having improved odor and/or taste characteristics.

14. Composition according to claim 12, wherein said composition is a mixture of chemical variants of an antibiotic compound and produced by a single culture.
